Europäisches Patentamt

**European Patent Office**

Office européen des brevets

(19)

(11) Publication number: **0 167 210 B1**

# EUROPEAN PATENT SPECIFICATION

(45) Date of publication of patent specification: **24.04.91**    (51) Int. Cl.⁵: **C11D 3/50, C11D 3/48**

(21) Application number: 85201028.9

(22) Date of filing: 28.06.85

(54) **Para-dichlorobenzene-free lavatory cleansing blocks.**

(30) Priority: **06.07.84 GB 8417345**

(43) Date of publication of application:
**08.01.86 Bulletin 86/02**

(45) Publication of the grant of the patent:
**24.04.91 Bulletin 91/17**

(84) Designated Contracting States:
**AT BE CH DE FR GB IT LI NL SE**

(56) References cited:
EP-A- 0 014 979
EP-A- 0 101 402
WO-A-83/03056
DE-A- 937 640
DE-A- 3 331 995

(73) Proprietor: **UNILEVER NV**
**Burgemeester s'Jacobplein 1 P.O. Box 760**
**NL-3000 DK Rotterdam(NL)BE CH DE FR IT LI**
**NL SE AT**

Proprietor: **UNILEVER PLC**
**Unilever House Blackfriars P.O. Box 68**
**London EC4P 4BQ(GB)GB**

(72) Inventor: **Clarke, David Ellis**
**31, Brookhurst Road**
**Bromborough Wirral Merseyside L63**
**0EH(GB)**
Inventor: **Joy, Bryan Stuart**
**24, Kingsbury West Kirby**
**Wirral Merseyside L48 6ES(GB)**

(74) Representative: **Kan, Jacob Hendrik, Dr. et al**
**Unilever N.V. Patent Division P.O. Box 137**
**NL-3130 AC Vlaardingen(NL)**

## Description

This invention relates to lavatory cleansing blocks and to a process for making them. More particularly, this invention relates to the manufacture of cleansing blocks which are intended for use as a cleansing and deodorizing block contained in an open device which is hung below the rim of toilet bowls, by which the block will be activated by the flush water and effect its cleaning action each time the toilet bowl is flushed with the flush water from the cistern. These so-called "lavatory rim blocks" are not in constant contact with water, as distinct from the so-called "in-cistern" type cleansing blocks which are immersed in the flush water cistern of lavatory bowls, and hence will have different requirements as to structure, rate of wear, cracking, mushing and swelling properties, foam properties and perfume generation.

Conventional lavatory rim blocks generally contain para-dichlorobenzene (PDCB) in fairly high proportions, not only as part of the perfume component but also as a structuring aid and satisfactory PDCB-containing blocks can be manufactured without difficulty by casting a melt.

FR-A-2 050 577 discloses lavatory cleansing blocks comprising 45-70 parts by weight of PDCB, 5-30 parts by weight of sodium tetrapropylene benzene sulphonate, 5-15 parts by weight of sodium lauryl ether sulphate 1-7 parts by weight of sodium triphosphate, 1-5 parts by weight of chlorinated tri-sodium phosphate and 1-5 parts by weight of perfume, which are prepared by grinding and sieving the powdery materials, mixing them with a fluid to give a paste, which is extruded and cut into lengths.

US-A-3 943 243 discloses lavatory cleansing blocks comprising 10-65% by weight of sodium bisulphite and 90-35% by weight of para-dichlorobenzene, prepared by melting and casting or by compacting the solid components.

Concern is growing, however, in both Government and Environmentalist circles about the possible hazards of chlorinated hydrocarbon in sewage waters and pressure is mounting in a number of countries for legislation to be enacted limiting or even banning the use of PDCB in lavatory rim blocks.

Although alternative structuring aids to PDCB in blocks made by casting a melt are known, these are generally detrimental to either the wear or foaming properties of the block, particularly for those containing a high level of perfume.

The invention now provides a process for preparing an improved lavatory rim block having a high level of perfume, which is free from para-dichlorobenzene. Such blocks cannot be satisfactorily made by the conventional method of casting a melt in a mould.

PDCB-free lavatory cleansing blocks are known in the art.

GB-A-2 061 996 discloses PDCB-free lavatory cleansing blocks of the in-cistern type containing high amounts of nonionic surface-active agents and gelling agents, which are prepared by melting the nonionic, dispersing the remainder of the ingredients and casting the resultant melt/dispersion into moulds. These blocks are unsatisfactory for use as lavatory rim blocks due to their high gelling/swelling tendency and poor foaming properties.

GB-A 2 021 143 discloses PDCB-free lavatory cleansing blocks of the in-cistern type which are prepared by forming a mixture of particulate ingredients and compressing the mixture to form a tablet. Tablets of this type are unsatisfactory for use as lavatory rim blocks, since the force of the flush water impinging upon the tablet would tend to cause the tablet to disintegrate within the device, thereby shortening its lifetime.

EP-A-101,402 also discloses PDCB-free lavatory cleansing blocks of the in-cistern type. The blocks consist of two incompatible compositions and are manufactured by extruding the compositions and then combining them to form a single tablet.

WO-A-83/03056 discloses on page 17, under B, a PDCB-free lavatory cleansing block containing 6.5% by weight of a perfume composition. It is not indicated how the block can be manufactured or how it is used.

It has now been found that an improved PDCB-free cleansing lavatory rim block having a high level of perfume can be prepared from a carefully balanced composition of ingredients which are mixed to form a dough, which is then extruded and cut into lengths.

The PDCB-free lavatory rim block of the invention is made by preparing a dough comprising essentially from 40-90%, preferably from 50-80% by weight of an anionic surfactant, 5-55%, preferably 10-45% by weight of a filler and 5-15% by weight of an oily liquid perfume, the weight ratio between the anionic surfactant and the filler, if electrolytic, being greater than 2. This dough is then extruded as a single composition and is cutted into blocks of suitable lengths.

A preferred perfume level is from 7-12% by weight.

Blocks of this formula cannot be satisfactorily produced utilizing the conventional casting technique.

Anionic surfactants which can be used in the present invention include for example the alkali metal alkyl

2

substituted benzene sulphonates, alkali metal long chain alkyl sulphates, alkali metal ether sulphates derived from long chain alcohols and alkyl phenols, alkali metal alkane sulphonates, alkali metal olefin sulphonates and alkali metal sulphosuccinates, of which the sodium salts are preferably used.

Desirably the anionic surfactant used in the block should have a relatively low perfume solubility and should only slowly absorb water to optimize the ratio of oily liquid and lamellar phases to solid and inverted hexagonal phases.

Preferred anionic surfactants are sodium $C_9$-$C_{14}$ alkyl benzene sulphonates, sodium $C_{11}$-$C_{20}$ olefin sulphonates, sodium $C_{11}$-$C_{20}$ alkane sulphonates and sodium long chain $C_{10}$-$C_{14}$ alkyl sulphates or mixtures thereof, sodium alkyl benzene sulphonates being particularly preferred as the main surfactant component.

In addition, other types of surfactants, such as nonionic surfactants including the ethoxylated nonionic compounds, fatty acid alkanolamides and amine oxides, may be incorporated as desired, but only in amounts not exceeding 20% by weight, preferably less than 10% by weight, since they could easily tend to affect the foaming properties and rate of wear of the blocks.

The filler used in the composition of the lavatory rim block can be an electrolyte, such as sodium sulphate, sodium carbonate and the various phosphates , such as sodium triphosphate, hexametaphosphate, pyrophosphate and orthophosphate; or and inert material, such as calcite, clay and urea.

Background studies have indicated that a high electrolyte content tends to suppress the solubility of the block to such an extent that there is insufficient active present in the bowl after flushing to generate a stable foam. Hence, if an electrolyte is used as the filler, it is necessary to have a high ratio of surfactant to filler levels in the block, i.e. a high proportion of surfactant and a low proportion of electrolyte.

It has been found that the anionic surfactant/ electrolyte filler weight ratio should be greater than about 2 in order to avoid excessive swelling in use and to provide adequate foam generation and stability.

On the other hand the anionic surfactant/filler ratio is not critical if an inert filler is used.

Taking the above conditions into consideration, the ingredients can be easily mixed to prepare a dough of suitable consistency which can be satisfactorily extruded and cut into lengths to form blocks having the desired properties.

Depending on the anionic surfactant raw material as supplied from different manufacturers, it may be necessary to adjust the consistency of the dough, i.e. soften the dough before extrusion, by incorporating a small amount of water, usually not more than about 1.5% by weight.

If such is the case, it is preferred to premix the added water with the required level of perfume to maintain a coherent dough; otherwise non-dispersible lumps will form and the dough cannot be satisfactorily extruded.

For extruding the dough no specially designed equipment is necessary. Extrusion can be carried out in simple conventional extrusion equipment normally used for manufacturing soap bars.

The lavatory rim blocks formed in accordance with the invention may also contain non-surfactant nonionic polymeric materials, such as polyethylene glycols and minor ingredients, such as dyes, germicides, fungicides, bleaches, opacifiers and the like as desired. Blocks of the invention will suitably have a weight of from 20-150 grams, preferably from 50-80 grams.

The invention will now be illustrated by way of the following Examples.


EXAMPLES

Lavatory rim blocks were prepared from each of the following compositions listed in the Table below by mixing the ingredients to form a dough, which was then extruded in a conventional soap-extrusion equipment and cut into lengths having a weight of about 50 grams.

## TABLE

| Ingredients | % by weight in block | | | | | | | | |
|---|---|---|---|---|---|---|---|---|---|
| | 1 | 2 | 3 | 4 | A | B | C | D | E |
| Marlon[R] A 390 1) | – | – | – | – | 36 | – | 37 | – | – |
| Nansa[R] HS 85 2) | 80 | 60 | 60 | 60 | – | 36 | – | 37 | 54 |
| Anhydrous $Na_2SO_4$ | 12 | – | – | 16 | – | – | 55 | 55 | 36 |
| Anhydrous $Na_5P_3O_{10}$ | – | – | – | – | 54 | 52 | – | – | – |
| Calcite | – | 32 | – | – | – | – | – | – | – |
| Clay | – | – | 32 | – | – | – | – | – | – |
| Urea | – | – | – | 16 | – | – | – | – | – |
| Citron 141 perfume | 8 | 8 | 8 | 8 | 10 | 10 | 8 | 8 | 10 |
| Water | – | – | – | – | – | 2 | – | – | – |

1) Marlon A 390 is sodium $C_{10}$-$C_{13}$ alkyl benzene sulphonate of mean molecular weight 240, supplied by the Chem. Werke Hulst GmbH. (contains 10-15% $Na_2SO_4$ as impurities).

2) Nansa HS 85 is sodium $C_9$-$C_{13}$ alkyl benzene sulphonate of mean molecular weight 235, supplied by Marchon Company (contains 10-15% $Na_2SO_4$ as impurities).

Blocks 1-4 are within the invention and Blocks A-E are outside the invention, which were prepared and examined for comparison purposes.

- Block 1 gave excellent foam and perfume generation and stability throughout its in-use life. Storage properties were also excellent.
- Blocks 2-4 gave good foam and perfume generation and stability throughout their in-use life.
- Blocks A and B outside the invention tended to gain weight and swell in use. Foam generation and stability were poor.
- Block C outside the invention, whilst satisfactory initially, rapidly softened on storage until eventually a complete layer of mush formed on the surface.
- Blocks D and E outside the invention, whilst also satisfactory initially, became slighly hard on storage. In use Blocks C, D and E tended to swell, in some cases to such an extent that they came out of the cages of the hanging device. As with Blocks A and B foam generation and stability were poor.

Replacement of $Na_2SO_4$ by $Na_2CO_3$ gave blocks with equally poor foaming properties.

## Claims

1. Process for preparing a lavatory rim cleansing block which is free from para-dichlorobenzene characterized by preparing a dough comprising:
   from 40 to 90% by weight of an anionic surfactant;
   from 5 to 55% by weight of an inert or electrolyte
   filler; and
   from 5 to 15% by weight of an oily liquid perfume, the weight ratio between the anionic surfactant and the filler, if electrolytic, being greater than 2;
   extruding said dough as a single composition and cutting it into blocks of suitable lengths.

2. A process according to claim 1 wherein the dough comprises from 7 to 12% of the perfume.

3. A process according to claim 1 or 2 wherein the dough comprises from 50 to 80% of the anionic surfactant.

4. A process according to any one of the preceding claims wherein the dough comprises from 10 to 45%

EP 0 167 210 B1

of the filler.

5. A process according to any one of the preceding claims wherein the anionic surfactant comprises $C_9$-$C_{14}$ alkylbenzene sulphonate.

6. A process according to any one of the preceding claims wherein the dough further comprises a nonionic surfactant in an amount of less than 10% by weight.

7. A process according to any one of the preceding claims wherein water in an amount of less than 1.5% by weight is premixed with the perfume.

8. Use of the extruded block prepared by the process according to any one of the preceding claims, as a lavatory rim cleansing block.

**Revendications**

1. Procédé pour préparer un bloc de nettoyage de bord de cuvettes de toilettes ne contenant pas de para-dichlorobenzène, caractérisé par le fait qu'on prépare une pâte comprenant:
   de 40 à 90% en masse d'un agent tensio-actif anionique;
   de 5 à 55% en masse d'un agent de remplissage inerte ou électrolytique; et
   de 5 à 15% en masse d'un parfum liquide huileux, le rapport en masse entre l'agent tensio-actif anionique et l'agent de remplissage, s'il est électrolytique, étant supérieur à 2; en ce qu'on extrude ladite pâte sous forme d'une seule composition et en ce q'on découpe cette pâte en blocs de longueurs appropriées.

2. Un procédé selon la revendication 1 dans lequel la pâte comprend de 7 à 12% du parfum.

3. Un procédé selon la revendication 1 ou 2, dans lequel la pâte comprend de 50 à 80% de l'agent tensio-actif anionique.

4. Un procédé selon l'une des revendications précédentes dans lequel la pâte comprend de 10 à 45% de l'agent de remplissage.

5. Un procédé selon l'une des revendications précédentes dans lequel l'agent tensio-actif anionique comprend du $C_9$-$C_{14}$ alkylbenzène sulfonate.

6. Un procédé selon l'une des revendications précédentes, dans lequel la pâte comprend en plus un agent tensio-actif non ionique dans une quantité inférieure à 10% en masse.

7. Un procédé selon l'une des revendications précédentes, dans lequel l'eau dans une quantité inférieure à 1,5 % en masse, est préalablement mélangée au parfum.

8. L'utilisation du bloc extrudé, préparé par le procédé selon l'une des revendications précédentes, en tant que bloc de nettoyage de bord de cuvettes de toilettes.

**Ansprüche**

1. Verfahren zur Herstellung eines Reinigungsblocks für den Toilettenrand, der frei ist von Paradichlorbenzol, gekennzeichnet durch Herstellung eines Teigs, der
   40 bis 90 Gew.-% eines anionischen, oberflächenaktiven Materials,
   5 bis 55 Gew.-% eines inerten oder Elektrolyt-Füllstoffes, und
   5 bis 15 Gew.-% eines öligen, flüssigen Parfüms umfaßt, wobei das Gewichtsverhältnis zwischen dem anionischen, oberflächenaktiven Material und dem Füllstoff, wenn dieser elektrolytisch ist, größer als 2 ist;
   Extrudieren des Teigs als eine einzige Zusammensetzung und Schneiden von dieser in Blöcke geeigneter Länge.

5

2. Verfahren nach Anspruch 1, worin der Teig 7 bis 12 % des Parfüms umfaßt.

3. Verfahren nach Anspruch 1 oder 2, worin der Teig 50 bis 80 % des anionischen, oberflächenaktiven Materials umfaßt.

4. Verfahren nach einem der vorhergehenden Ansprüche, worin der Teig 10 bis 45 % des Füllstoffes umfaßt.

5. Verfahren nach einem der vorhergehenden Ansprüche, worin das anionische, oberflächenaktive Material $C_9$-$C_{14}$ Alkylbenzolsulfonat umfaßt.

6. Verfahren nach einem der vorhergehenden Ansprüche, worin der Teig weiterhin ein nicht-ionisches, oberflächenaktives Material in einer Menge von weniger als 10 Gew.-% umfaßt.

7. Verfahren nach einem der vorhergehenden Ansprüche, worin Wasser in einer Menge von weniger als 1,5 Gew.-% mit dem Parfüm vorgemischt wird.

8. Verwendung des extrudierten, gemäß dem Verfahren nach einem der vorhergehenden Ansprüche hergestellten Blocks als Reinigungsblock für den Toilettenrand.